# EUROPÄISCHE PATENTSCHRIFT

(11) **EP 3 058 897 B1**
(45) Veröffentlichungstag und Bekanntmachung des Hinweises auf die Patenterteilung: **12.07.2017**
(21) Anmeldenummer: 16152649.6
(22) Anmeldetag: 25.01.2016
(51) Int. Cl.: A61F 2/38

(54) **ENDOPROTHESE FÜR DIE KNIEGELENKARTHRODESE**
ENDOPROSTHESIS FOR KNEE JOINT ARTHRODESIS
ENDOPROTHESE POUR ARTHRODESE DU GENOU

(30) Priorität: 19.02.2015 DE 102015102391
(43) Veröffentlichungstag der Anmeldung: 24.08.2016
(73) Patentinhaber: implantcast GmbH, 21614 Buxtehude (DE)
(72) Erfinder: Saß, Jens, 21614 Buxtehude (DE); Prof. Dr. Gosheger, Georg, 48157 Münster (DE); Wensing, Martin, 48329 Havixbeck (DE); Borgert, Sebastian, 48653 Coesfeld (DE); Zscheile, Axel, 48341 Altenberge (DE)
(74) Vertreter: Raffay & Fleck

(56) Entgegenhaltungen:
- WO-A1-2010/025704
- GB-A- 2 002 638
- US-A1- 2009 171 463
- US-A1- 2014 025 172

## Beschreibung

Die Erfindung betrifft eine Endoprothese für die Kniegelenkarthrodese.

Die Kniegelenkarthrodese, d.h. die operative Versteifung des Kniegelenkes mit einem metallischen Implantat, einer Endoprothese, wird regelmäßig z.B. nach mehrfachem Versagen einer Kniegelenkendoprothese, bei einem Knochentumor, bei Knocheninfektionen, bei einem Trauma und bei neurologischen Erkrankungen wie der Poliomyelitis, der sogenannten Kinderlähmung, durchgeführt.

In der Vergangenheit wurden hierzu unterschiedliche metallische Implantate eingesetzt, die eine vollständige Versteifung des Kniegelenks und Fixierung von Oberschenkel zu Unterschenkel an der Position des ursprünglichen Kniegelenks in einem vorgegebenen und festen Winkel bewirkten. Bisherige Systeme, wie z.B. dasjenige gemäß WO 2010/025704 A1, bestehen dabei in der Regel aus Schaftsystemen, die in den verbleibenden Unter- und Oberschenkelknochen (Tibia und Femur) verankert und dann mit Kopplungshülsen fixiert, d.h. vollständig, dauerhaft versteift werden. Allen vorbekannten Lösungsansätzen ist dabei die Tatsache gemein, dass aufgrund der dauerhaften Versteifung des Kniegelenks in einem vorgegebenen Winkel die funktionellen Ergebnisse der damit verfolgten Vorgehensweise sehr schlecht sind.

Allgemein ist die Bewegungsfreiheit eines betroffenen Patienten mit in einem festen Winkel dauerhaft versteiftem Kniegelenk erheblich eingeschränkt. So kann ein betroffener Patient mit einem vorbekannten Implantat insbesondere nur mit Problemen in Verkehrsmitteln, wie Flugzeugen, Autos oder dergleichen, transportiert werden. Weiterhin zeigen sich auch erhebliche Einschränkungen der sexuellen Aktivität bei fehlender Beugemöglichkeit des Knies. Aufgrund der Kraftübertragung auf die umliegenden Gelenke kommt es zudem nicht selten zu sekundärarthrotischen Veränderungen im Bereich der unteren Lendenwirbelsäule sowie der Hüft- und Sprunggelenke.

In der US 2014/0025174 A1 ist ein verriegelbares Knieimplantat offenbart, mit welchem eine Schwenkposition des mit dem Implantat gebildeten künstlichen _Kniegelenks verriegelt werden kann. Dieses Implantat ist für die Verwendung in der Revisionsendoprothetik vorgesehen, wenn also noch stabilisierende Elemente des natürlichen Gelenkapparates, wie z.B. eine Restgelenkkapsel, muskuläre Strukturen oder ein Restbandapparat vorhanden sind, die auf das Gelenk wirkende Kräfte mit abfangen. Das dort offenbarte Knieimplantat ist dagegen nicht für einen totalen Knochenersatz geeignet, sondern wird stets nur als Oberflächenersatz zu verwenden sein. Für den totalen Knochenersatz wurden bisher lediglich Systeme verwendet, wie Sie vorstehend beschrieben wurde und z.B. in der bereits genannten WO 2010/025704 A1 offenbart sind.

Hier soll mit der Erfindung Abhilfe geschaffen werden, indem eine Endoprothese für die Kniegelenkarthrodese geschaffen wird, die einerseits die gewünschte und stabile Versteifung gewährleistet, so insbesondere beim Laufen eine stabile, in Streckung versteifte Führung des Beines ergibt, andererseits aber auch eine Veränderung der Beugestellung des Unterschenkels relativ zum Oberschenkel gestattet.

Diese Aufgabe wird erfindungsgemäß gelöst mit einer Endoprothese für die Kniegelenkarthodese mit den Merkmalen des Anspruchs 1. Vorteilhafte Weiterbildungen der erfindungsgemäßen Endoprothese sind in den abhängigen Ansprüchen 2 bis 11 angegeben.

Erfindungsgemäß hat die neuartige Endoprothese für die Kniegelenkarthrodese also folgende Bestandteile:
a. ein Femurteil, das eine Femuranschlussstruktur für die feste Verbindung mit dem distalen Ende des Femurs (Oberschenkelknochens) aufweist,
b. ein Tibiateil, welches eine Tibiaanschlussstruktur für die feste Verbindung mit dem proximalen Ende der Tibia (des Schienbeinknochens) aufweist,
c. einen an dem Femurteil ausgebildeten femurseitigen Kopplungsabschnitt,
d. einen an dem Tibiateil ausgebildeten tibiaseitigen Kopplungsabschnitt, wobei der femurseitige Kopplungsabschnitt und der tibiaseitige Kopplungsabschnitt miteinander um eine Prothesenschwenkachse, die in Einbaulage der Endoprothese als Knieersatz im Wesentlichen horizontal von medial nach lateral verläuft und somit in ihrer Lage im Wesentlichen der Lage der Schwenkachse des natürlichen Knies entspricht, relativ zueinander verschwenkbar verbunden sind,
e. wenigstens ein Riegelelement, welches zwischen einer Riegelstellung, in der es den femurseitigen Kopplungsabschnitt und den tibiaseitigen Kopplungsabschnitt miteinander in einer vorgebbaren Schwenkstellung starr verriegelt, und einer Freigabestellung, in der ein Verschwenken des femurseitigen Kopplungsabschnittes und des tibiaseitigen Kopplungsabschnittes relativ zueinander um die Prothesenschwenkachse ermöglicht ist, hin und her bewegbar ist,
f. ein Halte- und Rückstellmittel, welches das Riegelelement mit einer Haltekraft in die Riegelstellung zwingt und in dieser hält, und
g. einen Entriegelungsmechanismus, mittels dem das Riegelelement entgegen der Haltekraft aus der Riegelstellung in die Freigabestellung verlagerbar ist.

Die Erfindung zeichnet sich nun dadurch aus, dass bei der Endoprothese einer der Kopplungsabschnitte, femurseitiger Kopplungsabschnitt oder tibiaseitiger Kopplungsabschnitt, einen an beiden Stirnseiten verschlossenen, im Querschnitt kreisförmigen Hohlzylinderabschnitt aufweist, der mit seiner Zylinderlängsachse entlang der Prothesenschwenkachse orientiert ist. Bei dieser Ausgestaltung der erfindungsgemäßen Endoprothese weist ferner der andere Kopplungsabschnitt, tibiaseitiger Kopplungsabschnitt oder femurseitiger Kopplungsabschnitt, einen parallel zu der Prothesenschwenkachse verlaufenden, hohlkehlenförmigen Lagerabschnitt zur um die Zylinderlängsachse verdrehbaren Aufnahme des Hohlzylinderabschnittes sowie zudem eine quer zu der Prothesenschwenkachse verlaufende Anschlagwand auf, welche Anschlagwand einen stirnseitigen Anschlag für den Hohlzylinderabschnitt bildet. Weiterhin sind bei dieser Ausgestaltungsvariante in der Anschlagwand an unterschiedlichen Positionen wenigstens zwei parallel zu der Prothesenschwenkachse geführte Aufnahmebohrungen vorgesehen und umfasst das Riegelelement eine in einem Innenraum des Hohlzylinders in der Zylinderlängsrichtung verlagerbar angeordnete Kolbenplatte an der wenigstens ein in Zylinderlängsrichtung verlaufender, von dieser in Richtung der Anschlagwand vorstehender Riegelzapfen exzentrisch angeordnet ist. Dieser Riegelzapfen durchragt mit seinem freien Ende eine Öffnung in der der Anschlagwand zugewandten Stirnseite der Hohlzylinderabschnittes und kann in einer relativen Schwenkposition der beiden Kopplungsabschnitte, in der er mit einer der Aufnahmebohrungen in der Anschlagwand fluchtet, in diese durch Bewegen der Kolbenplatte in Zylinderlängsrichtung ein- bzw. aus dieser ausfahren.

Das Wesentliche und Besondere an der neuartigen und erfindungsgemäßen Endoprothese für die Kniegelenksarthrodese ist also, dass das Femurteil und das Tibiateil über die jeweils beteiligten Kopplungsabschnitte nicht starr in einem unveränderbar vorgegebenen Winkel zueinander festgelegt werden, sondern dass hier eine Verschwenkmöglichkeit gegeben ist. Anders als bei echten Gelenkendoprothesen, die die Gelenkfunktion des natürlichen Gelenks ersetzen sollen, ist diese Verschwenkmöglichkeit allerdings nicht frei verfügbar, um eine natürliche Gelenkfunktion abzubilden. Vielmehr ist bei der erfindungsgemäßen Endoprothese vorgesehen, dass diese in vorgegebenen Schwenkstellungen verriegelt und so eine starre und steife Verbindung zwischen Femur und Tibia in einem vorgegebenen Winkel herstellt und einhält. Hierzu ist das Riegelelement vorgesehen, welches durch das Halte- und Rückstellmittel in einer Riegelstellung gehalten wird, so dass die erfindungsgemäße Endoprothese in einer Normalstellung sich in einer verriegelten und im Hinblick auf die Winkelstellung zwischen Tibia und Femur definiert versteiften Position befindet. Allerdings kann eben das Riegelelement mit Hilfe des Entriegelungsmechanismus aus der Riegelstellung gelöst und kann dann ein anderer Schwenkwinkel zwischen Tibia und Femur eingestellt werden. Ein entsprechendes Beugen oder Strecken zum Verändern des Schwenkwinkels wird der Patient aufgrund der weitreichenden Zerstörung im Bereich des ehemaligen Kniegelenkes typischerweise nicht aus eigener Kraft mit dem Muskelapparat des betroffenen Beines vornehmen. Er wird den entsprechenden Schwenkwinkel vielmehr extern, also z.B. unter Zuhilfenahme seiner Arme und Hände einstellen und dann durch Loslassen des Entriegelungsmechanismus dafür sorgen, dass das Riegelelement durch das Halte- und Rückstellmittel in der nun eingestellten Schwenkposition wieder verriegelt und damit Tibia und Femur in diesem Beugewinkel relativ zueinander fixiert und versteift.

In der erfindungsgemäßen Ausgestaltung der Kopplungsabschnitte ist eine besonders einfache auf die Prothesenschwenkachse beschränkte Relativbewegungsmöglichkeit geschaffen, für die mit Hilfe des Riegelzapfens, der in entsprechende Aufnahmebohrungen der Anschlagwand einfahren kann, eine sichere und zuverlässige Verriegelungsmöglichkeit in vorgegebenen Schwenkpositionen gegeben ist.

Das Besondere an dem Entriegelungsmechanismus ist dabei, dass dieser von außerhalb des Beines zu bedienen ist. Insbesondere müssen dafür keine Bestandteile der Endoprothese freiliegen und durch die Haut geführt werden, sondern der betroffene Patient kann den Entriegelungsmechanismus des im Inneren des Körpers gelegenen Implantates, der erfindungsgemäßen Endoprothese, durch die Haut und das aufliegende Gewebe hindurch von außen bedienen. Grundsätzlich käme hier beispielsweise ein Betätigungsschalter oder Knopf in Betracht, der unterhalb der Haut und ggf. unterhalb weiteren Gewebes angeordnet ist und den der Patient durch z.B. Drücken betätigen kann. Bevorzugt wird hier aber eine Ausgestaltungsvariante, bei der das Riegelelement als Bestandteil des Entriegelungsmechanismus ein Magnetelement aufweist, auf welches mit einem von außen in die Nähe dieses Magnetelementes geführten Magneten eine Kraft aufbringbar ist, mittels derer das Riegelelement entgegen der Haltekraft aus der Riegelstellung in die Freigabestellung verlagerbar ist. Bei einer solchen Lösung kann der Patient mit Hilfe eines von ihm mitgeführten, ausreichend starken Magneten die Entriegelung der Endoprothese vornehmen, indem er diesen Magneten in den Bereich des so überbrückten Knies bringt und dadurch durch die Haut und das darunter liegende Gewebe eine entsprechend hohe Magnetkraft auf das Riegelelement aufbringt, um dieses aus der Riegelstellung in die Freigabestellung zu bewegen.

In Ruhe kann der Patient also, z.B. mit dem Magneten, durch den Weichteilmantel das Riegelelement innerhalb der Endoprothese lösen, die dann eine Beweglichkeit des Kniegelenkes erlaubt. Damit kann der Patient zum Beispiel im Flugzeug oder Kino mit einem abgewinkelten Kniegelenk sitzen. Er kann auch sexuelle Handlungen in Beugestellung des betroffenen Beines durchführen. Beim Fahrradfahren kann das Bein als Passivbein mitschwingen, wenn der Patient durch entsprechende Dauerbetätigung des Riegelelements (also Halten desselben in der Freigabestellung) für eine fortwährende Verschwenkmöglichkeit der beiden Teile, Tibiateil und Femurteil, der Endoprothese sorgt.

Um einerseits eine besonders sichere und feste Verriegelung der beiden Elemente Tibiateil und Femurteil in vorgegebener Schwenkposition relativ zueinander zu gewährleisten und andererseits zugleich mehrere Schwenkpositionen ansteuern zu können, können an der Kolbenplatte, wie dies eine vorteilhafte Weiterbildung der Erfindung vorsieht, mehrere, insbesondere vier, regelmäßig und exzentrisch angeordnete, in Zylinderlängsrichtung verlaufende Riegelzapfen festgelegt sein, wobei diese entsprechend positionierte Öffnungen in der der Anschlagwand zugewandten Stirnseite des Hohlzylinderabschnittes durchragen und wobei in der Anschlagwand Aufnahmebohrungen in der Anzahl der Riegelzapfen entsprechender Anzahl und in einer der Verteilung der Riegelzapfen entsprechenden Verteilung bzw. Anordnung vorgesehen sind. Auf diese Weise wird also eine mit mehreren Riegelzapfen bewirkte und somit besonders feste und stabile sowie belastbare Verriegelung geschaffen, die auch beispielsweise beim Gehen mit in Streckstellung versteiftem Beim die entsprechende Belastung mit dem Körpergewicht des Patienten aushält, ohne Gefahr zu laufen, hier etwa zu entriegeln und zu einem ungewollten Abknicken des Beines im Kniebereich zu führen.

Wenn mit der vorstehend genannten Lösung, die die Kolbenplatte mit einschließt, zugleich der Entriegelungsmechanismus wie vorstehend beschrieben durch das mit dem Riegelelement verbundene Magnetelement realisiert wird, kann, wie es eine vorteilhafte Weiterbildung der Erfindung vorsieht, die Kolbenplatte das Magnetelement aufweisen oder insgesamt durch das Magnetelement gebildet sein.

Um die Kolbenplatte in dem Innenraum des Hohlzylinders verdrehsicher zu führen, kann diese mit Vorteil gemäß einer möglichen Weiterbildung der Erfindung wenigstens eine von ihrem Rand nach innen ragende Ausnehmung aufweisen, die mit einer an einer den Innenraum des Hohlzylinderabschnittes begrenzenden Wand ausgebildeten, in den Innenraum hineinragenden und in Zylinderlängsrichtung verlaufenden sowie im Querschnitt der Form der Ausnehmung angepassten Rippe zusammenwirkt, um somit eine verdreh- und auch verkippsichere Längsführung der Kolbenplatte zu erhalten.

Gemäß einer weiteren vorteilhaften Weiterbildung der erfindungsgemäßen Endoprothese kann der wenigstens eine Riegelzapfen jedenfalls an seinem freien Ende aus einem magnetisierbaren oder aus einem magnetischen Material gebildet sein und können in den Aufnahmebohrungen je ein Haltemagnet angeordnet sein, der das Halte- und Rückstellmittel bildet. Dieser Haltemagnet übt auf den ihm gegenüberliegenden Riegelzapfen eine magnetische Anziehungskraft aus, so dass der Riegelzapfen dann, wenn er in einer Position in Flucht mit der Aufnahmebohrung liegt, in diese Aufnahmebohrung hineingezogen wird in die Riegelstellung, in der er die Endoprothese in einer vorgegebenen Schwenkstellung sicher verriegelt.

Mit Vorteil kann, wie dies eine besondere Weiterbildung der Erfindung vorsieht, der Hohlzylinderabschnitt an dem femurseitigen Kopplungsabschnitt ausgebildet sein, der Lagerabschnitt an dem tibiaseitigen Kopplungsabschnitt angeordnet sein.

Insbesondere sind bei der erfindungsgemäßen Endoprothese die beiden Kopplungsabschnitte miteinander zwar verschwenkbar aber ansonsten nicht relativ verlagerbar verbunden. Hierfür kann gemäß einer vorteilhaften Weiterbildung der Erfindung ein entlang der Prothesenschwenkachse geführter, die beiden Kopplungsabschnitte verbindender Dreh- und Schraubbolzen verwendet werden. Unter einem Dreh- und Schraubbolzen im Sinne dieser Erfindung wird ein solcher Schraubbolzen verstanden, der durch Verschrauben eines an seinem Schaft ausgebildeten Außengewindes mit einem entsprechenden Innengewinde in einer Bolzenaufnahme festgelegt werden kann, der jedoch auch über einen glattwandigen Schaftabschnitt verfügt, der sodann gegenüber einem glattwandigen Abschnitt einer Öffnung, durch die der Bolzen hindurchgeführt ist, eine Drehachse bzw. ein Drehlager bildet.

Um bei der erfindungsgemäßen Endoprothese sicherzustellen, dass nicht etwa Beugewinkel zwischen Femur und Tibia eingestellt werden können, die anatomisch keinen Sinn ergeben, vielmehr eine Gefährdung des Patienten darstellen könnten, können mit Vorteil Schwenkwinkelbegrenzungsstrukturen an den zusammenwirkenden Kopplungsabschnitten vorhanden sein, die ein relatives Verschwenken von Tibiateil zu Femurteil hinsichtlich eines vorgegebenen Schwenkwinkels limitieren.

Schließlich können gemäß vorteilhafter Weiterbildungen der Erfindung die Femuranschlussstruktur ein Femurschaft und die Tibiaanschlussstruktur ein Tibiaschaft sein. Derartige Schaftanschlüsse sind hinreichend bekannt und haben sich als besonders geeignet zum Festlegen am distalen Femurende bzw. am proximalen Tibiaende erwiesen, indem diese Schaftstrukturen in den Markraum des jeweiligen Röhrenknochens eingebracht und dort beispielsweise mittels Knochenzementes festgelegt werden.

Weitere Vorteile und Merkmale der erfindungsgemäßen Endoprothese für die Kniegelenkarthrodese ergeben sich aus der nachfolgenden Beschreibung eines möglichen Ausführungsbeispiels anhand der beigefügten Figuren. Dabei zeigen:
- Fig. 1: ein Ausführungsbeispiel einer erfindungsgemäßen Endoprothese für die Kniegelenkarthrodese verriegelt in einer Streckstellung;
- Fig. 2: die Endoprothese gemäß Fig. 1, jedoch in Beugestellung verbracht und darin verriegelt;
- Fig. 3: die Endoprothese aus Fig. 1 bzw. 2 in einer in die jeweiligen Einzelteile zerlegten Explosionsdarstellung;
- Fig. 4: in einer dreidimensionalen Ansicht den tibiaseitigen Kopplungsabschnitt der Endoprothese gemäß dem Ausführungsbeispiel;
- Fig. 5: eine Seitenansicht des in Fig. 4 gezeigten Tibiaabschnittes von der in Fig. 4 links dargestellten Seite her;
- Fig. 6: eine dreidimensionale Ansicht des femurseitigen Kopplungsabschnittes der Endoprothese nach dem Ausführungsbeispiel;
- Fig. 7: eine Seitenansicht des femurseitigen Kopplungsabschnittes aus Fig. 6 von der in Fig. 6 links dargestellten Seite her;
- Fig. 8: in einer dreidimensionalen Darstellung das mit vier Riegelzapfen versehene Riegelelement des Ausführungsbeispiels der erfindungsgemäßen Endoprothese; und
- Fig. 9: einen Magneten für die Entriegelung des Riegelelementes von außerhalb des betroffenen Beines eines mit der Endoprothese gemäß dem Ausführungsbeispiel versorgten Patienten her.

In den Figuren ist ein Ausführungsbeispiel für eine erfindungsgemäße Endoprothese für die Kniegelenkarthrodese dargestellt, welches Ausführungsbeispiel nachfolgend zum besseren Verständnis und zur weiteren Erläuterung der Erfindung beschrieben werden wird. Die Figuren sind dabei als Prinzipdarstellungen zu verstehen, die die wesentlichen Elemente des Ausführungsbeispiels zeigen und keinesfalls als vollständige Konstruktionszeichnungen zu verstehen sind.

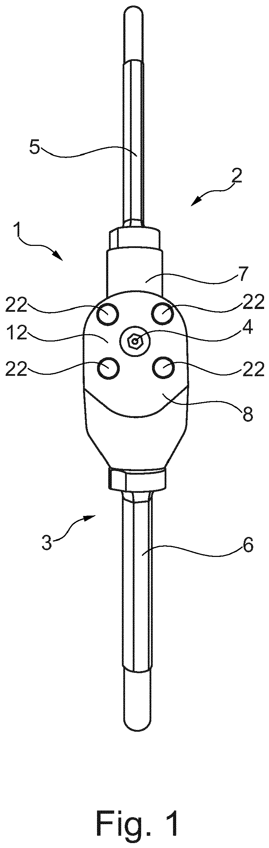

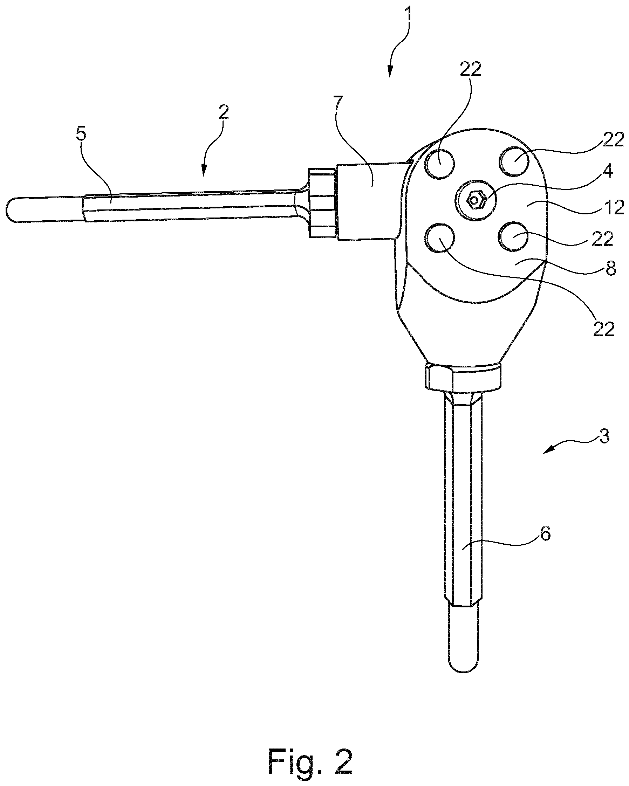

Das in den Figuren gezeigte Ausführungsbeispiel zeigt eine mögliche Gestaltungsvariante einer erfindungsgemäßen Endoprothese, die in den Figuren allgemein mit der Bezugsziffer 1 bezeichnet ist. Diese Endoprothese 1 hat ein Femurteil 2 und ein Tibiateil 3, die mit Hilfe eines Dreh- und Schraubbolzens 4 miteinander verbunden sind. Dabei sind bei der erfindungsgemäßen Endoprothese 1 das Femurteil 2 und das Tibiateil 3 um eine Prothesenschwenkachse, die entlang der Längsachse des eingeschraubten Dreh- und Schraubbolzens 4 verläuft, relativ zueinander verschwenkbar. Auf diese Weise kann die Endoprothese 1 beispielsweise in einer in Fig. 1 gezeigten gestreckten Stellung eingestellt und darin fixiert (siehe hierzu später) werden, aber auch alternativ in einer wie in Fig. 2 gezeigten gebeugten Stellung.

Am Femurteil 2 der Endoprothese 1 ist ein Femurschaft 5 angeordnet und festgelegt, der in an sich bekannter Weise im Markkanal des distalen Femurendes festgelegt, typischerweise mit Knochenzement einzementiert wird, um das Femurteil 2 mit dem Femur des Patienten fest zu verbinden. In gleicher Weise ist an dem Tibiateil 3 ein Tibiaschaft 6 festgelegt, der in den Markkanal des proximalen Tibiaendes eingeführt und dort festgelegt wird, um das Tibiateil fest mit der Tibia des Patienten zu verbinden. Das Femurteil weist ferner einen femurseitigen Kopplungsabschnitt 7 auf; das Tibiateil 3 ist mit einem tibiaseitigen Kopplungsabschnitt 8 gebildet. Diese beiden Kopplungsabschnitte 7, 8 sind mittels des Dreh- und Schraubbolzens 4 miteinander verbunden und können relativ zueinander um die Prothesenschwenkachse verschwenkt werden.

In einer implantierten Situation verläuft dabei die Prothesenschwenkachse im Wesentlichen horizontal und von medial nach lateral und entspricht in ihrer Lage dabei im Wesentlichen der Hauptschwenkachse des natürlichen Kniegelenkes (ohne dabei auch die im natürlichen Kniegelenk möglichen Schwenkbewegungen um eine Vertikalachse mit abbilden zu können).

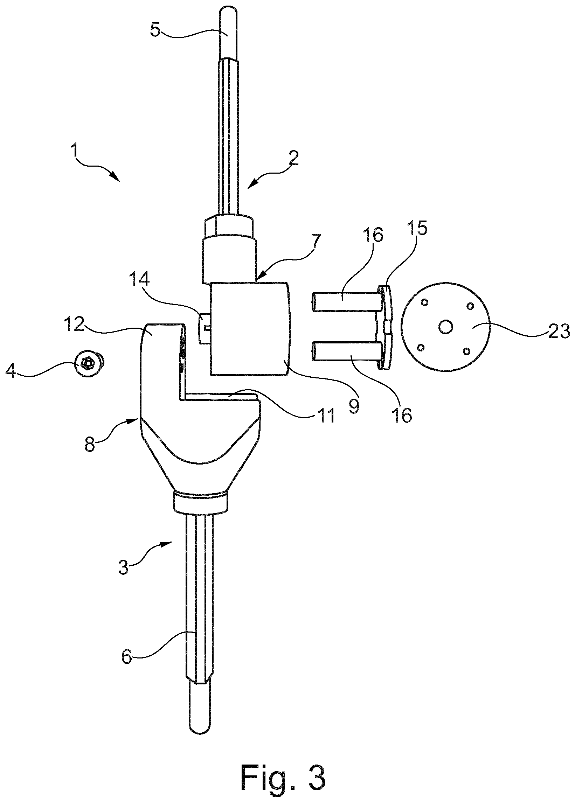

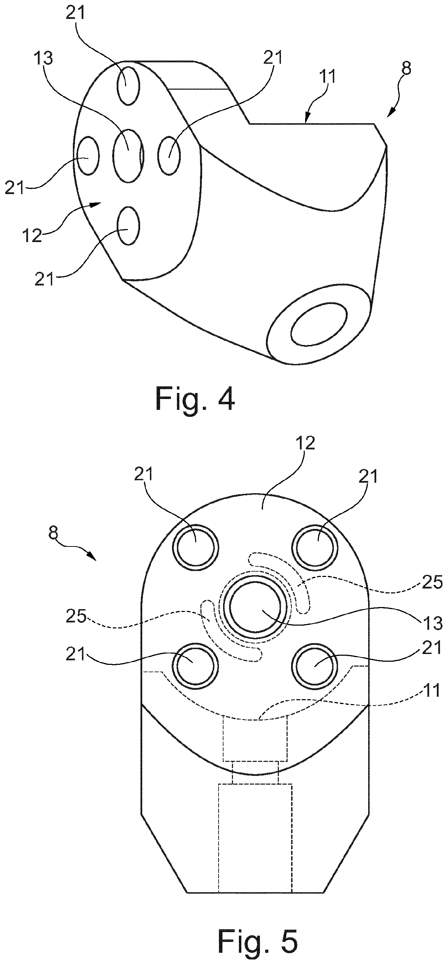

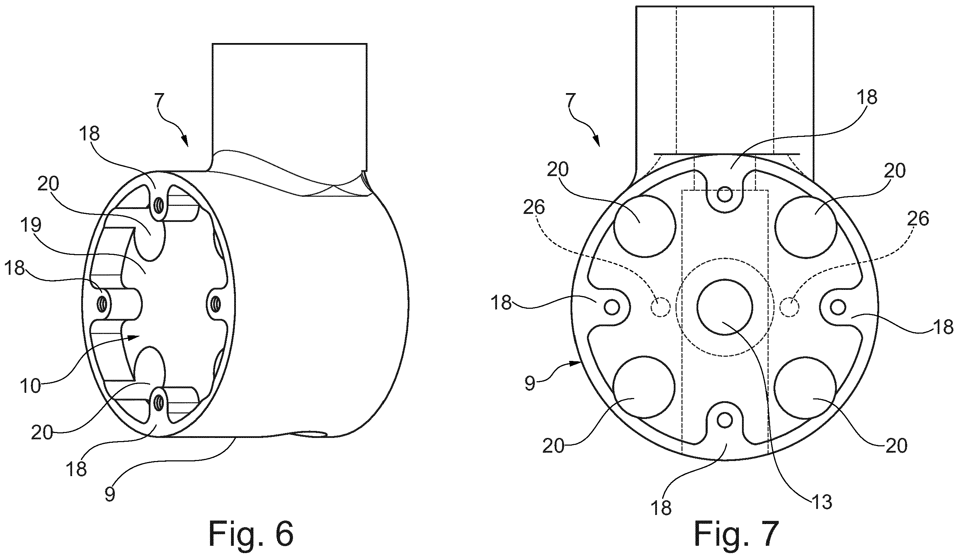

In der in Fig. 3 gezeigten Explosionsdarstellung ist gut zu erkennen, dass der femurseitige Kopplungsabschnitt 7 einen Hohlzylinderabschnitt 9 aufweist, der einen kreisförmigen Durchmesser und in seinem Inneren einen Innenraum 10 (vgl. Fig. 6) aufweist. Dieser Hohlzylinderabschnitt 9 wird im zusammengefügten Zustand der Endoprothese 1, also bei mit den jeweiligen Kopplungsabschnitten 7, 8 zusammengesetzten Femurteil 2 und Tibiateil 3 in einem hohlkehlenförmigen Lagerabschnitt 11 des tibiaseitigen Kopplungsabschnittes 8 aufgenommen, welcher hohlkehlenförmige Lagerabschnitt 11 eine der Außenkontur des Hohlzylinderabschnittes 9 entsprechende Negativform aufweist. An diesem Lagerabschnitt 11 schließt sich als weiterer Bestandteil des tibiaseitigen Kopplungsabschnittes 8 eine Anschlagwand 12 an. In dieser befindet sich eine zentrale Durchführungsbohrung 13 (vgl. Fig. 4 und 5), in die von der an den Lagerabschnitt 11 angrenzenden Seite her ein Drehzapfen 14, der an der der Anschlagwand 12 zugewandten Stirnseite des Hohlzylinderabschnittes 9 angeformt ist, eingeführt liegt und durch die zum Verbinden der beiden Kopplungsabschnitte 7, 8 der Dreh- und Schraubbolzen 4 hindurchgeführt und mit einem Außengewinde in ein in einer zentralen Bohrung in dem Drehzapfen 14 eingearbeitetes Innengewinde eingeschraubt wird, um auf diese Weise die beiden Kopplungsabschnitte 7, 8 und damit das Femurteil 2 und das Tibiateil 3 miteinander zu verbinden, jedoch ein Verschwenken um die Prothesenschwenkachse zu erlauben.

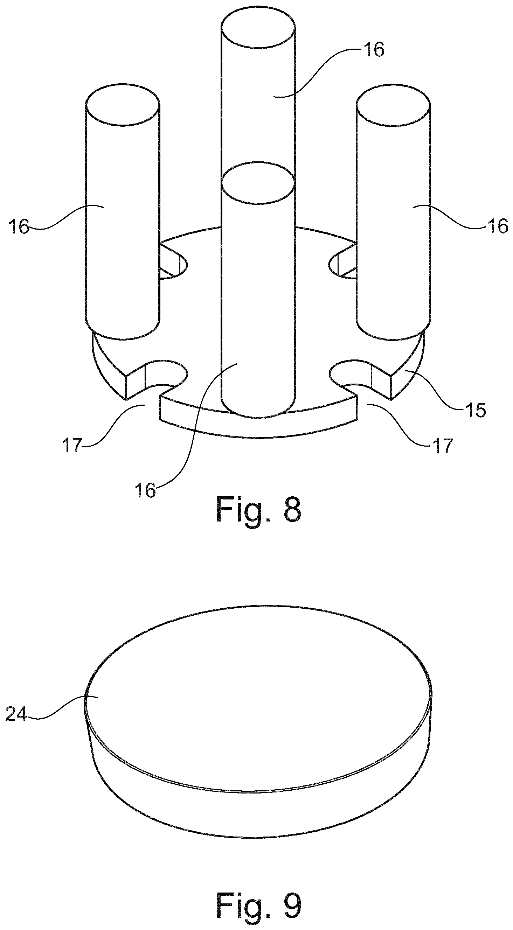

In den Innenraum 10 des Hohlzylinderabschnittes 9 eingesetzt ist ein Riegelelement, welches aus einer Kolbenplatte 15 mit daran angeformten, parallel in Längsrichtung des Zylinderabschnittes 9 orientierten Riegelzapfen 16 gebildet ist. Wie insbesondere Fig. 8 zeigt, sind an der Kolbenplatte 15 exzentrisch insgesamt vier Riegelzapfen 16 angeordnet, wobei diese auf einem gleichen Durchmesser und äquidistant zueinander verteilt platziert sind. Zwischen den Positionen, an denen die Riegelzapfen 16 an der Kolbenplatte 15 angeformt sind, sind jeweils von der Außenseite der Kolbenplatte 15 her in Richtung deren Mitte geführte, U-förmige Ausnehmungen 17 (insgesamt vier solcher Ausnehmungen 17) geführt. Diese umgreifen im zusammengefügten Zustand nach innen in den Innenraum 10 des Hohlzylinderabschnittes 9 vorstehende, in der Zylinderlängsrichtung verlaufende Längsrippen 18, so dass die Kolbenplatte 15 insgesamt verdreh- und verkippsicher im Innenraum 10 des Hohlzylinderabschnittes 9 geführt ist.

Die Riegelzapfen 16 durchragen in der Stirnwand 19 des Hohlzylinderabschnittes 9 gebildete Öffnungen 20. Die Kolbenplatte 15 mit den daran angeformten Riegelzapfen 16 ist in dem Hohlraum 10 des Hohlzylinderabschnittes 9 in der Zylinderlängsrichtung verschiebbar angeordnet. Ist dabei die Kolbenplatte 15 gegen die Stirnwand 19 gedrückt und schlägt an dieser an, so stehen die Riegelzapfen 16 mit ihren freien Enden über den Hohlzylinderabschnitt 19 über und greifen, wenn diese in Flucht zu den Riegelzapfen 16 positioniert sind, in Aufnahmeöffnungen 21 ein, so dass sie den femurseitigen Kopplungsabschnitt 7 und den tibiaseitigen Kopplungsabschnitt 8 in einer relativen Schwenkposition zueinander verriegeln und dass mit der Endoprothese 1 versorgte Knie in einer eingenommenen Schwenkposition (z.B. einer gestreckten Position gemäß Fig. 1 bzw. einer gebeugten Position gemäß Fig. 2) starr und fest verriegeln.

In den Aufnahmeöffnungen 21 sind bündig mit der dem Lagerabschnitt 11 abgewandten Außenseite der Anschlagwand 12 abschließend kreiszylinderförmige Permanentmagnete 22 eingebracht und dort festgelegt. Diese Permanentmagnete, bei denen es sich z.B. um Neodym N 48-Magnete handeln kann, wirken auf die freien Enden der Riegelzapfen 16 eine magnetische Anziehungskraft aus und zwingen diese in einer Normalstellung in die Aufnahmebohrungen 21 hinein, halten sie somit in einer Verriegelungsstellung.

Auf der der Anschlagwand 12 abgewandten Stirnseite ist der Hohlzylinderabschnitt 9 mit einer in Fig. 3 zu erkennenden Abdeckplatte 23 verschlossen, die mittels vier Schrauben, die in entsprechende Gewindebohrungen in den freiliegenden Stirnseiten der Längsrippen 18 eingeschraubt sind, an dem Hohlzylinderabschnitt 9 festgelegt ist. Diese Abdeckplatte 23 bildet darüber hinaus einen zweiten Anschlag für die Kolbenplatte 15, die sich in dem Innenraum 10 mit einem entsprechenden Hub zwischen der Stirnwand 19 und der Abdeckplatte 23 in Richtung der Zylinderlängsachse und somit auch in Richtung der Prothesenschwenkachse hin und her bewegen kann.

Um nun das Riegelelement in Form der Kolbenplatte 15 mit den daran angeformten Riegelzapfen 16 aus seiner Riegelstellung, in der die freien Enden der Riegelzapfen 16 in die Aufnahmeöffnungen 21 eingreifen und dort von den Permanentmagneten 22 gehalten werden, in eine Freigabestellung zu bewegen, in der die freien Enden der Riegelzapfen 16 vollständig aus den Aufnahmeöffnungen 21 herausgenommen sind, wird ein Permanentmagnet, beispielsweise ein Neodym N 48-Magnet, verwendet, wie dieser beispielsweise in Fig. 9 gezeigt und mit dem Bezugszeichen 24 bezeichnet ist. Dieser Permanentmagnet 24 wird von außen an das mit der erfindungsgemäßen Endoprothese 1 versorgte Knie herangeführt und ist mit seiner Magnetkraft ausreichend stark, um durch Haut und Gewebe und die Abdeckplatte 23 hindurch die magnetische bzw. magnetisierbare Kolbenplatte 15 anzuziehen. Insbesondere ist der Permanentmagnet 24 dabei mit seiner Magnetkraft stärker als die in den Aufnahmeöffnungen 21 angeordneten Permanentmagnete 22, so dass trotz der Haltekraft der Permanentmagnete 22 die Kolbenplatte 15 mit daran angeformten Riegelzapfen 16 unter Überwindung dieser Haltekraft aus der Riegelstellung in die Freigabestellung gezogen wird, nun die Endoprothese 1 und damit das mit dieser versorgte Bein in einer anderen Winkelstellung eingestellt werden kann. Wird der Permanentmagnet 24 dann wieder aus der unmittelbaren Nähe der Endoprothese 1 entfernt, ziehen die Permanentmagnete 22 in den Aufnahmeöffnungen 21 die freien Enden der Riegelzapfen 16 wieder in die Aufnahmeöffnungen 21 hinein, so dass die Riegelzapfen 16 mit ihren freien Enden in die Aufnahmeöffnungen 21 einfahren, wenn diese fluchtend zu den Riegelzapfen 16 ausgerichtet sind. Damit wird dann die Endoprothese 1 in einer anderen Winkelstellung wiederum verriegelt.

Schließlich ist in den Figuren auch zu erkennen, dass Strukturen zur Begrenzung des Schwenkwinkels, um den die Elemente Femurteil 2 und Tibiateil 3 relativ zueinander um die Prothesenschwenkachse verschwenkt werden können, vorgesehen sind. Diese Elemente sind auf der dem Lagerabschnitt 11 zugewandten Innenseite der Anschlagwand 12 eingebrachte, entlang eines einen maximalen Schwenkwinkel beschreibenden Kreisabschnittes jeweils geführte, einander gegenüberliegende Führungsnuten 25 sowie an der der Anschlagwand 12 zugewandten äußeren Stirnseite des Hohlzylinderabschnittes 9 in einander diametral gegenüberliegenden Positionen angeformte Führungszapfen 26, die im zusammengefügten Zustand von femurseitigem Kopplungsabschnitt 7 mit tibiaseitigem Kopplungsabschnitt 8 in den Führungsnuten 25 laufen und entsprechend an den Enden dieser Führungsnuten den Schwenkwinkel beschränkende Anschläge erfahren.

Aus der vorstehenden Beschreibung eines Ausführungsbeispiels ist noch einmal deutlich geworden, welche besonderen Vorteile die erfindungsgemäße Endoprothese 1 für die Kniegelenkarthrodese mit sich bringt. Die hier erstmals geschaffene Möglichkeit, eine Endoprothese für die Kniegelenkarthrodese in unterschiedlichen Schwenkstellungen einstellen und blockieren zu können, verschafft dem betroffenen Patienten ein deutlich höheres Maß an Lebensqualität, da er beispielsweise bei Kinobesuchen oder im Flugzeug das betroffene Bein durch Entriegeln des Riegelelementes und Verstellen der Endoprothese 1 in ihrem Schwenkwinkel anwinkeln und in diesem Winkel wieder verriegeln kann. Indem der Patient einen Permanentmagneten beispielsweise mit einer um das betroffene Knie angelegten Manschette dauerhaft so im Bereich der Endoprothese platziert, dass dieser die Kolbenplatte 15 und mit ihr die Riegelzapfen 16 in der Entriegelungsstellung, in der die Riegelzapfen 16 nicht mehr in den Aufnahmeöffnungen 21 liegen, hält, kann zudem eine passive Verschwenkbarkeit des betroffenen Beines im Kniebereich geschaffen werden, so dass z.B. mit einer entsprechenden Befestigung des Fußes des betroffenen Beines an einer Pedale der betroffene Patient Fahrradfahren kann, wobei das mit der Endoprothese 1 versorgte Bein passiv mitschwingt, die Vortriebskraft von dem mit einem natürlichen oder einem prothetisch ersetzten funktionsfähigen Kniegelenk versehenen Bein aufgebracht wird.

### Bezugszeichenliste

- 1: Endoprothese
- 2: Femurteil
- 3: Tibiateil
- 4: Dreh- und Schraubbolzen
- 5: Femurschaft
- 6: Tibiaschaft
- 7: femurseitiger Kopplungsabschnitt
- 8: tibiaseitiger Kopplungsabschnitt
- 9: Hohlzylinderabschnitt
- 10: Innenraum
- 11: Lagerabschnitt
- 12: Anschlagwand
- 13: Durchführungsbohrung
- 14: Drehzapfen
- 15: Kolbenplatte
- 16: Riegelzapfen
- 17: Ausnehmung
- 18: Längsrippe
- 19: Stirnwand
- 20: Öffnung
- 21: Aufnahmeöffnung
- 22: Permanentmagnet
- 23: Abdeckplatte
- 24: Permanentmagnet
- 25: Führungsnut
- 26: Führungszapfen

## Patentansprüche

1. Endoprothese (1) für die Kniegelenkarthrodese mit
a. einem eine Femuranschlussstruktur (5) für die feste Verbindung mit dem distalen Ende des Femurs aufweisenden Femurteil (2),
b. einem eine Tibiaanschlussstruktur(6) für die feste Verbindung mit dem proximalen Ende der Tibia aufweisenden Tibiateil (3),
c. einem an dem Femurteil (2) ausgebildeten femurseitigen Kopplungsabschnitt (7),
d. einem an dem Tibiateil (3) ausgebildeten tibiaseitigen Kopplungsabschnitt (8),
wobei der femurseitige Kopplungsabschnitt (7) und der tibiaseitige Kopplungsabschnitt (8) miteinander um eine Prothesenschwenkachse, die in Einbaulage der Endoprothese (1) als Knieersatz im Wesentlichen horizontal von medial nach lateral verläuft und somit in ihrer Lage im Wesentlichen der Lage der Schwenkachse des natürlichen Knies entspricht, relativ zueinander verschwenkbar verbunden sind,
e. wenigstens einem Riegelelement (15, 16), welches zwischen einer Riegelstellung, in der es den femurseitigen Kopplungabschnitt (7) und den tibiaseitigen Kopplungsaschnitt (8) miteinander in einer vorgebbaren Schwenkstellung starr verriegelt, und einer Freigabestellung, in der ein Verschwenken des femurseitigen Kopplungsabschnittes (7) und des tibiaseitigen Kopplungsabschnittes (8) relativ zueinander um die Prothesenschwenkachse ermöglicht ist, hin und her bewegbar ist,
f. einem Halte- und Rückstellmittel (22), welches das Riegelelement (15, 16) mit einer Haltekraft in die Riegelstellung zwingt und in dieser hält, und
g. einem Entriegelungsmechanismus, mittels dessen das Riegelelement (15, 16) entgegen der Haltekraft aus der Riegelstellung in die Freigabestellung verlagerbar ist,
**dadurch gekennzeichnet, dass** einer der Kopplungsabschnitte, femurseitiger Kopplungsabschnitt (7) oder tibiaseitiger Kopplungsabschnitt (8), einen an beiden Stirnseiten verschlossenen, im Querschnitt kreisförmigen Hohlzylinderabschnitt (9) aufweist, der mit seiner Zylinderlängsachse entlang der Prothesenschwenkachse orientiert ist, und dass der andere Kopplungsabschnitt, tibiaseitiger (8) Kopplungsabschnitt oder femurseitiger Kopplungsabschnitt (7), einen parallel zu der Prothesenschwenkachse verlaufenden, hohlkehlenförmigen Lagerabschnitt (11) zur um die Zylinderlängachse verdrehbaren Aufnahme des Hohlzylinderabschnitts (9) und eine quer zu der Prothesenschwenkachse verlaufende Anschlagwand (12), die einen stirnseitigen Anschlag für den Hohlzylinderabschnitt (9) bildet, aufweist, wobei in der Anschlagwand (12) an unterschiedlichen Positionen wenigstens zwei parallel zu der Prothesenschwenkachse geführte Aufnahmebohrungen (21) vorgesehen sind, wobei das Riegelelement (15, 16) eine in einem Innenraum (10) des Hohlzylinderabschnitts (9) in der Zylinderlängsrichtung verlagerbar angeordnete Kolbenplatte (15) umfasst, an der wenigstens ein in Zylinderlängsrichtung verlaufender, von der Kolbenplatte (15) in Richtung der Anschlagwand (12) vorstehender Riegelzapfen (16) exzentrisch angeordnet ist, wobei der Riegelzapfen (16) mit seinem freien Ende eine Öffnung (20) in der der Anschlagwand (12) zugewandten Stirnseite (19) des Hohlzylinderabschnitts (9) durchragt und, in einer relativen Schwenkposition der beiden Kopplungsabschnitte (7, 8), in der er mit einer der Aufnahmebohrungen (21) in der Anschlagwand (12) fluchtet, in diese er durch Bewegen der Kolbenplatte (15) in Zylinderlängsrichtung ein- und aus dieser ausfahren kann.

2. Endoprothese (1) nach Anspruch 1, **dadurch gekennzeichnet, dass** an der Kolbenplatte (15) mehrere, insbesondere vier, regelmäßig und exzentrisch angeordnete, in Zylinderlängsrichtung verlaufende Riegelzapfen (16) festgelegt sind, dass diese entsprechend positionierte Öffnungen (20) in der der Anschlagwand (12) zugewandten Stirnseite (19) des Hohlzylinderabschnitts (9) durchragen und dass in der Anschlagwand (12) Aufnahmebohrungen (21) in der Anzahl der Riegelzapfen (16) entsprechender Anzahl und in einer der Verteilung der Riegelzapfen (16) entsprechender Verteilung vorgesehen sind.

3. Endoprothese (1) nach einem der vorhergehenden Ansprüche, **dadurch gekennzeichnet, dass** das Riegelelement (15, 16) als Bestandteil des Entriegelungsmechanismus ein Magnetelement aufweist, auf welches mit einem von außen in die Nähe dieses Magnetelements geführten Magneten (24) eine Kraft aufbringbar ist, mittels derer das Riegelelement (15, 16) entgegen der Haltekraft aus der Riegelstellung in die Freigabestellung verlagerbar ist.

4. Endoprothese (1) nach Anspruch 3, **dadurch gekennzeichnet, dass** die Kolbenplatte (15) das Magnetelement aufweist oder dieses bildet.

5. Endoprothese (1) nach einem der vorhergehenden Ansprüche, **dadurch gekennzeichnet, dass** die Kolbenplatte (15) wenigstens eine von ihrem Rand nach innen ragende Ausnehmung (17) aufweist und dass an einer den Innenraum (10) des Hohlzylinderabschnittes (9) begrenzenden Wand wenigstens eine im Querschnitt der Form der Ausnehmung (17) angepasste, in den Innenraum (10) hineinragende und in der Zylinderlängsrichtung verlaufende Rippe (18) vorgesehen ist, die für eine verdreh- und verkippsichere Führung der Kolbenplatte (15) mit der Ausnehmung (17) in Eingriff steht.

6. Endoprothese (1) nach einem der vorhergehenden Ansprüche, **dadurch gekennzeichnet, dass** der Riegelzapfen (16) jedenfalls an seinem freien Ende aus einem magnetisierbaren oder aus einem magnetischen Material gebildet ist und dass in den Aufnahmebohrungen (21) je ein Haltemagnet (22) angeordnet ist, der das Halte- und Rückstellmittel bildet.

7. Endoprothese nach einem der vorhergehenden Ansprüche, **dadurch gekennzeichnet, dass** der Hohlzylinderabschnitt (9) an dem femurseitigen Kopplungsabschnitt (7), der Lagerabschnitt (11) an dem tibiaseitigen Kopplungsabschnitt (8) angeordnet ist.

8. Endoprothese (1) nach einem der vorhergehenden Ansprüche, **dadurch gekennzeichnet, dass** die beiden Kopplungsabschnitte (7, 8) mittels eines entlang der Prothesenschwenkachse geführten Dreh- und Schraubbolzens (4) um die Prothesenschwenkachse relativ zueinander verdrehbar verbunden sind.

9. Endoprothese nach einem der vorhergehenden Ansprüche, **gekennzeichnet, durch** Schwenkwinkelbegrenzungsstrukturen (25, 26) an den zusammenwirkenden Kopplungsabschnitten (7, 8), die ein relatives Verschwenken von Tibiateil (3) zu Femurteil (2) hinsichtlich eines vorgegebenen Schwenkwinkels limitieren.

10. Endoprothese nach einem der vorhergehenden Ansprüche, **dadurch gekennzeichnet, dass** die Femuranschlussstruktur (5) ein Femurschaft ist.

11. Endoprothese nach einem der vorhergehenden Ansprüche, **dadurch gekennzeichnet, dass** die Tibiaanschlussstruktur(6) ein Tibiaschaft ist.

## Claims

1. An endoprosthesis (1) for the knee joint arthrodesis with
a. a femur part (2) having a femur connection structure (5) for a fixed connection with the distal end of the femur,
b. a tibial part (3) having a tibia connection structure (6) for fixed connection with the proximal end of the tibia;
c. a femur-side coupling portion (7) formed on the femur part (2),
d. a tibia-side coupling portion (8) formed on the tibia part (3),
wherein the femur-side coupling section (7) and the tibia-side coupling section (8), are pivotally connected relative to one another about a prosthesis pivoting axis, which extends essentially horizontally from medial to lateral as knee replacements in the mounting position of the endoprosthesis (1) and thus essentially corresponds to the position of the pivot axis of the natural knee,
e. at least one locking element (15, 16) movable back and forth between a locking position in which it rigidly locks the femur-side coupling portion (7) and the tibia-side coupling portion (8) with one another in a predeterminable pivot position, and a release position, enabling pivoting of the femur-side coupling portion (7) and the tibia-side coupling section (8) relative to one another around the prosthesis pivot axis,
f. a holding and resetting means (22), which forces the locking element (15, 16) with a holding force into the locking position and holds it therein,
and
g. a release mechanism by means of which the locking element (15, 16) is displaceable against the retaining force from the locking position into the release position,
**characterised in that** one of the coupling portions, the femur-side coupling section (7) or the tibia-side coupling section (8), has a hollow-cylinder portion (9) which is closed on both ends and has a circular cross-section, which is oriented along the prosthesis pivoting axis with its cylinder longitudinal axis, and the other coupling portion, i.e. the tibia coupling portion (8) or the femur-side coupling portion (7), includes a bearing portion (11), in the form of a fillet, extending parallel to the prosthesis pivoting axis, for receiving the hollow cylinder portion (9) in a rotating manner around the cylinder longitudinal axis, and a stop wall (12) extending perpendicular to the prosthesis pivoting axis and forming a front stop for the hollow cylinder portion (9), wherein at least two locating bores (21) parallel to the prosthesis pivoting axis are provided in the stop wall (12) at different positions, wherein the locking element (15, 16) include a piston plate (15) provided mobile in an internal space (10) of the hollow cylinder portion (9) in the cylinder longitudinal direction, on which at least one locking spigot (16) is provided eccentrically, extending in the cylinder longitudinal direction, protruding from the piston plate (15) in the direction of the stop wall (12), wherein the locking spigot (16) penetrates with its free end into an opening in the front side (19) of the hollow cylinder portion (9) facing the stop wall (12) and in a relative pivoting position of both coupling portions (7, 8) in which it is flush with one of the locating bores (21) in the stop wall (12), in which it can protrude and into it which it can retract by moving the piston plate (15) in the cylinder longitudinal direction.

2. The endoprosthesis (1) according to claim 1, **characterised in that** several, in particular four locking spigots (16) extended in the cylinder longitudinal direction, provided at regular intervals and eccentrically, are fixed to the piston plate (15), that said penetrate through openings (20) placed accordingly, in the front side (19) of the hollow cylinder portion (9) facing the stop wall (12) and that locating bores (21) are provided in the stop wall (12), which bores are in a number corresponding to the number of the locking spigots (16) and with a distribution corresponding to the distribution of the locking spigots (16).

3. The endoprosthesis (1) according to one of the preceding claims, **characterised in that** the locking element (15, 16) has a magnetic element as integral part of the release mechanism, on which a force can be applied with a magnet (24) guided from the outside in the proximity of this magnet element, by means of which the locking element (15, 16) is displaceable against the retaining force from the locking position into the release position.

4. The endoprosthesis (1) according to claim 3, **characterised in that** the piston plate (15) has or forms the magnetic element.

5. The endoprosthesis (1) according to one of the preceding claims, **characterised in that** the piston plate (15) has at least one recess (17) protruding inwardly from its rim and that at least one rib (18) is provided, extending in the cylinder longitudinal direction, penetrating into the inside, adapted to the form of the recess (17) in its cross-section, at the wall delineating the inside (10) of the hollow cylinder portion (9), which rib is engaged in the recess (17) for guiding the piston plate (15) while preventing the latter from rotating or tipping.

6. The endoprosthesis (1) according to one of the preceding claims, **characterised in that** the locking spigot (16) consists of a magnetisable or of a magnetic material, respective at its free end and that a holding magnet (22) is provided respectively in the locating bores (21), which magnet forms the holding and resetting means.

7. The endoprosthesis according to one of the preceding claims, **characterised in that** the hollow cylinder portion (9) is provided at the femur-side coupling portion (7) and that the bearing portion (11) is provided at the tibia-side coupling portion (8).

8. The endoprosthesis (1) according to one of the preceding claims, **characterised in that** both coupling portions (7, 8) are connected to be able to rotate relative to one another around the prosthesis pivoting axis by means of a rotating and threaded bolt guided along the prosthesis pivoting axis.

9. The endoprosthesis according to one of the preceding claims, **characterised by** structures (25, 26) limiting the swing angle at the co-operating coupling portions (7, 8), which structures limit the relative swinging of the tibia part (3) with respect to the femur part (2) to respect a preset swing angle.

10. The endoprosthesis according to one of the preceding claims, **characterised in that** the femur connection structure (5) is a femoral shaft.

11. The endoprosthesis according to one of the preceding claims, **characterised in that** the tibia connection structure (6) is a tibial shaft.

## Revendications

1. Endoprothèse (1) pour arthrodèse du genou comprenant
a. une partie fémorale (2) ayant une structure (5) de connexion avec le fémur pour assurer une connexion fixe avec l'extrémité distale du fémur,
b. une partie tibiale (3) ayant une structure (6) de connexion avec le tibia pour assurer une connexion fixe avec l'extrémité proximale du tibia ;
c. une partie accouplement côté fémur (7) formée sur la partie fémur (2),
d. une partie accouplement côté tibia (8) formée sur la partie tibia (3),
où la partie accouplement côté fémur (7) et la partie accouplement côté tibia (8), sont reliées de manière pivotante l'une par rapport à l'autre autour d'un axe de pivotement de la prothèse, qui s'étend essentiellement horizontalement de la médiale à la latérale en tant que prothèses du genou dans la position de montage de l'endoprothèse (1) et correspond donc essentiellement à la position de l'axe de pivotement du genou naturel,
e. au moins un élément de verrouillage (15, 16) mobile en va-et-vient entre une position de verrouillage dans laquelle il verrouille rigidement la partie accouplement côté fémur (7) et la partie accouplement côté tibia (8) l'une avec l'autre dans une position de pivotement prédéfinissable, et une position de libération, permettant le pivotement de la partie accouplement côté fémur (7) et de la partie accouplement côté tibia (8) l'une par rapport à l'autre autour de l'axe de pivotement de la prothèse,
f. un moyen de maintien et de réinitialisation (22), qui force l'élément de verrouillage (15, 16) avec une force de maintien dans la position de verrouillage et le retient, et
g. un mécanisme de libération au moyen duquel l'élément de verrouillage (15, 16) est déplaçable contre la force de retenue de la position de verrouillage dans la position de libération,
**caractérisé en ce que** l'une des parties accouplement, la partie accouplement côté fémur (7) ou la partie accouplement côté tibia (8) présente une partie cylindrique creuse (9) qui est fermée sur les deux extrémités et présente une section transversale circulaire, partie qui est orientée le long de l'axe de pivotement de la prothèse avec son axe longitudinal de cylindre, et l'autre partie accouplement, à savoir la partie accouplement côté tibia (8) ou la partie accouplement côté fémur (7), comprend une partie d'appui (11), sous forme de filet, s'étendant parallèlement à l'axe de pivotement de la prothèse, pour recevoir la partie cylindrique creuse (9) en rotation autour de l'axe longitudinal du cylindre, et une paroi de butée (12) s'étendant perpendiculairement à l'axe de pivotement de la prothèse et formant une butée avant pour la partie cylindrique creuse (9), dans laquelle au moins deux alésages de positionnement (21) parallèles à l'axe de pivotement de la prothèse sont prévus dans la paroi de butée (12) dans différentes positions, où l'élément de verrouillage (15, 16) comporte une plaque de piston (15) prévue mobile dans un espace interne (10) de la partie cylindrique creuse (9) dans la direction longitudinale du cylindre, sur lequel au moins un ergot de verrouillage (16) est disposé excentriquement, s'étendant dans la direction longitudinale du cylindre, faisant saillie de la plaque de piston (15) dans la direction de la paroi de butée (12), où l'ergot de verrouillage (16) pénètre avec son extrémité libre dans une ouverture sur le côté avant (19) de la partie cylindrique creuse (9) en regard de la paroi de butée (12) et dans une position de pivotement relative des deux parties accouplement (7, 8), position dans laquelle il est affleurant avec l'un des alésages de positionnement (21) dans la paroi de butée (12), dans laquelle il peut faire saillie et se rétracter en déplaçant la plaque de piston (15) dans la direction longitudinale du cylindre.

2. Endoprothèse (1) selon la revendication 1, **caractérisée en ce que** plusieurs, en particulier quatre ergots de verrouillage (16) s'étendant dans la direction longitudinale du cylindre, prévus à intervalles réguliers et excentriquement, sont fixées à la plaque de piston (15), que lesdits ergots pénètrent à travers des ouvertures (20) placées de manière adéquate, dans le côté avant (19) de la partie cylindrique creuse (9) faisant face à la paroi de butée (12) et **en ce que** des alésages de positionnement (21) sont prévus dans la paroi de butée (12), lesquels alésages sont en nombre correspondant au nombre des ergots de verrouillage (16) et avec une répartition correspondant à la répartition des ergots de verrouillage (16).

3. Endoprothèse (1) selon l'une quelconque des revendications précédentes, **caractérisée en ce que** l'élément de verrouillage (15, 16) possède un élément magnétique comme partie intégrante du mécanisme de libération, sur lequel une force peut être appliquée avec un aimant (24) guidé de l'extérieur à proximité de cet élément magnétique, au moyen duquel l'élément de verrouillage (15, 16) peut être déplacé contre la force de retenue depuis la position de verrouillage dans la position de libération.

4. Endoprothèse (1) selon la revendication 3, **caractérisée en ce que** la plaque de piston (15) possède ou forme l'élément magnétique.

5. Endoprothèse (1) selon l'une quelconque des revendications précédentes, **caractérisée en ce que** la plaque de piston (15) présente au moins un évidement (17) faisant saillie vers l'intérieur à partir de sa jante et qu'au moins une nervure (18) est prévue, s'étendant dans la direction longitudinale du cylindre, pénétrant dans l'intérieur, adapté à la forme de l'évidement (17) dans sa section transversale, à la paroi délimitant l'intérieur (10) de la partie cylindrique creuse (9), laquelle nervure est engagée dans l'évidement (17) pour guider la plaque de piston (15) tout en empêchant celle-ci de tourner ou de basculer.

6. Endoprothèse (1) selon l'une quelconque des revendications précédentes, **caractérisée en ce que** l'ergot de verrouillage (16) est constitué d'un matériau magnétisable ou magnétique, respectif à son extrémité libre et qu'un aimant de retenue (22) est prévu respectivement dans les alésages de positionnement (21), lequel aimant forme les moyens de maintien et de réinitialisation.

7. Endoprothèse selon l'une quelconque des revendications précédentes, **caractérisée en ce que** la partie cylindrique creuse (9) est prévue au niveau de la partie accouplement côté fémur (7) et **en ce que** la partie d'appui (11) est prévue au niveau de la partie accouplement côté tibia (8).

8. Endoprothèse (1) selon l'une quelconque des revendications précédentes, **caractérisée en ce que** les deux parties accouplement (7, 8) sont connectées pour pouvoir tourner l'une par rapport à l'autre autour de l'axe de pivotement de la prothèse au moyen d'un boulon rotatif et fileté guidé le long de l'axe de pivotement de la prothèse.

9. Endoprothèse selon l'une quelconque des revendications précédentes, **caractérisé par** des structures (25, 26) limitant l'angle de rotation au niveau des parties accouplement coopérantes (7, 8), lesquelles structures limitent le pivotement relatif de la partie tibia (3) par rapport à la partie fémur (2) pour respecter un angle de pivotement prédéterminé.

10. Endoprothèse selon l'une quelconque des revendications précédentes, **caractérisée en ce que** la structure de connexion avec le fémur (5) est un arbre fémoral.

11. Endoprothèse selon l'une quelconque des revendications précédentes, **caractérisée en ce que** la structure de connexion avec le tibia (6) est un arbre tibial.
